(19) Europäisches Patentamt
European Patent Office
Office européen des brevets

(11) Veröffentlichungsnummer: **0 111 325**
**B1**

# (12) EUROPÄISCHE PATENTSCHRIFT

(45) Veröffentlichungstag der Patentschrift: **27.01.88**

(51) Int. Cl.⁴: **C 07 C 175/00**

(21) Anmeldenummer: **83112376.5**

(22) Anmeldetag: **08.12.83**

## (54) Verfahren zur Herstellung von 13-cis-Retinsäure.

(30) Priorität: **10.12.82 US 448660**

(43) Veröffentlichungstag der Anmeldung: **20.06.84 Patentblatt 84/25**

(45) Bekanntmachung des Hinweises auf die Patenterteilung: **27.01.88 Patentblatt 88/4**

(84) Benannte Vertragsstaaten: **AT BE CH DE FR GB IT LI NL**

(56) Entgegenhaltungen:
**US - A - 4 051 174**

**JOURNAL OF THE CHEMICAL SOCIETY Nr. 15, 8. November 1965, pages 347/349, G. PATTENDEN et al.: "Synthesis of cis- and Di-cis-Polyenes by Reactions of the Wittig Type. A New Isomer of Vitamin A Acid".**

(73) Patentinhaber: **F. HOFFMANN-LA ROCHE & CO. Aktiengesellschaft, CH-4002 Basel (CH)**

(72) Erfinder: **Lucci, Robert, 306 Meadowbrook Road, Wickoff, N.J. (US)**

(74) Vertreter: **Lederer, Franz, Dr. et al, Van der Werth, Lederer & Riederer Patentanwälte Lucile-Grahn-Strasse 22, D-8000 München 80 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 111 325 B1

ACTORUM AG

**Beschreibung**

13-cis-Retinsäure (13-cis-Vitamin-A-Säure) ist ein wertvolles Arzneimittel im Hinblick auf seine Verwendung zur Behandlung von Akne. Dieses Isomer ist jedoch sehr schwer herzustellen. Pattenden und Weedon, J. Chem. Soc. (C), 1984–1997 (1968) haben ein Verfahren zur Herstellung von 13-cis-Retinsäure beschrieben, bei dem ein [3-Methyl-5-(2,6,6-trimethyl-1-cyclohexen-1-yl)-2,4-pentadienyl]-triphenylphosphoniumsalz und 5-Hydroxy-4-methyl-2(5H)-furanon zu einem Gemisch von cis-Isomeren der Retinsäure umgesetzt wird. Dieses Verfahren hat den Nachteil, dass die Ausbeute an dem Gemisch der cis-Isomeren der Retinsäure gering ist. Weiterhin ist es schwierig, dieses Isomerengemisch durch Isomerisierung in das spezifische Isomere, nämlich 13-cis-Retinsäure zu überführen. Dies ist insbesondere deshalb der Fall, weil die bei diesem Verfahren erhaltenen Isomeren cis-Konfiguration in 11- und 13-Stellung aufweisen. Es hat sich als sehr schwierig erwiesen, die 11-cis-Doppelbindung selektiv ohne Isomerisierung der 13-cis-Doppelbindung zu isomerisieren. Viele der angewandten Isomerisierungstechniken sind relativ unwirksam bei der selektiven Isomerisierung der 11-cis-Doppelbindung. Jede Isomerisierung vermindert die Ausbeute an 13-cis-Vitamin-A-Säure.

Die Synthese von 13-cis- und 11,13-di-cis-Retinsäure-Isomerengemischen ist auch in J. Org. Chem. C, 347–349 (1965) beschrieben.

Es bestand daher ein Bedürfnis nach einem Verfahren zur Herstellung von 13-cis-Retinsäure in hohen Ausbeuten und in reiner Form.

Erfindungsgemäss wurde gefunden, dass 13-cis-Retinsäure dadurch hergestellt werden kann, dass man

a) 5-Hydroxy-4-methyl-2(5H)-furanon mit einem Salz der Formel

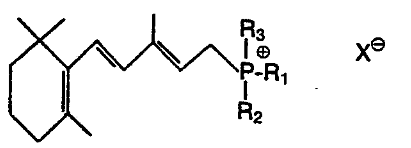

I

worin $R_1$, $R_2$ und $R_3$ Aryl oder Di-(nieder-alkyl)amino und X Halogen sind, in einem niederen Alkanol in Gegenwart eines Alkalimetallhydroxids bei einer Temperatur von −10 bis −50 °C umsetzt, und

b) das in Stufe a) erhaltene Reaktionsprodukt in einem organischen Lösungsmittel mit einer Verbindung oder einem Komplex von Rhodium oder Palladium, ausgenommen Palladium- oder Rhodiumphthalocyanin oder einer ein Cyanidion enthaltenden Palladium- oder Rhodiumverbindung behandelt.

Ein Aspekt der Erfindung betrifft die Umsetzung des Butenolids, 5-Hydroxy-4-methyl-2(5H)-furanon mit dem Wittigsalz der Formel I zu einem Gemisch, das 13-cis-Retinsäure und 11,13-cis-Retinsäure enthält. Die Reaktion wird unter den Bedingungen einer Wittigreaktion durchgeführt. Obwohl die Verbindung der Formel I jedes übliche Wittigsalz sein kann, ist Triphenylphosphoniumchlorid als Verbindung der Formel I bevorzugt.

Im nächsten Verfahrensschritt zur Herstellung der 13-cis-Retinsäure wird die 11,13-di-cis-Retinsäure entweder als isolierte Verbindung oder im Gemisch mit 13-cis-Retinsäure in Ausbeuten von über 90% in 13-cis-Retinsäure umgewandelt. Der bevorzugte Katalysator für diese Isomerisierung ist eine Palladiumverbindung, die durch Umsetzung von Palladium(II)nitrat mit einem Tri(nieder-alkyl)- oder Arylphosphin und Tri(nieder-alkyl)-amin erhalten wird. Bei Verwendung dieses bevorzugten Katalysators wird die Isomerisierung zu 13-cis-Retinsäure beinahe sofort in hohen Ausbeuten bewerkstelligt, ohne dass recyclisiert werden muss.

Es wurde weiterhin gefunden, dass bei Durchführung der Wittigreaktion bei Temperaturen von −10 bis −50 °C, vorzugsweise −20 bis −50 °C, insbesondere −30 bis −45 °C ein Gemisch von 13-cis- und 11,13-di-cis-Isomeren in Ausbeuten von 90% und mehr erhalten wird. Darüberhinaus verhindert die Anwendung dieser Temperaturen die Bildung von anderen Retinsäureisomeren als 13-cis- oder 11,13-di-cis-Retinsäure.

Bei dieser Reaktion wird im allgemeinen eine Base und ein inertes organisches Lösungsmittel angewandt. Das organische Lösungsmittel wird so gewählt, dass es bei den für die Wittigreaktion angewandten niedrigen Temperaturen nicht fest wird. Die Wahl eines besonderen inerten Lösungsmittels hängt infolgedessen von der Temperatur ab, bei der die Wittigreaktion durchgeführt wird. Unter den bevorzugten Lösungsmittel sind nieder-Alkanole wie Isopropanol, Äthanol und Methanol, wobei Isopropanol besonders bevorzugt ist. Erfindungsgemäss bevorzugte Basen sind Alkalimetallhydroxide wie Kaliumhydroxid, Natriumhydroxid und Lithiumhydroxid. Bei weitem die besten Resultate in Bezug auf Ausbeute und Produktqualität werden unter Verwendung von Kaliumhydroxid und Isopropanol erhalten. Bei der Durchführung dieser Reaktion ist es im allgemeinen bevorzugt, in einer Inertgasatmosphäre, vorzugsweise unter Stickstoff zu arbeiten.

Der Verfahrensschritt a) der vorliegenden Erfindung liefert ein Gemisch der 13-cis- und 11,3-di-cis-Isomeren. Dieses Gemisch kann gewünschtenfalls direkt in das 13-cis-Isomer umgewandelt werden, ohne Isolierung des 11,13-di-cis-Isomers. Andererseits kann das 11,13-di-cis-Isomer gewünschtenfalls aus dem Gemisch isoliert werden und in das 13-cis-Isomer umgewandelt werden. Wenn man das Gemisch trennen will, können die üblichen Techniken zur Trennung von Isomeren Anwendung finden. Eine bevorzugte Methode ist fraktionierte Kristallisation.

Im allgemeinen liefert die Wittigreaktion ein Gemisch, das etwa 70 bis etwa 90% 11,13-di-cis-Retinsäure und etwa 10 bis etwa 30% des 13-cis-Isomers enthält. Bei Anwendung niedriger Temperaturen, d.h. Temperaturen unterhalb −20 °C und vorzugsweise −30 bis −50 °C erhält man ein Gemisch von Retinsäuren in einer Ausbeute von

mindestens 90%, bezogen auf das als Ausgangsmaterial verwendete Butenolid.

Im nächsten Schritt des erfindungsgemässen Verfahrens wird entweder die 11,13-di-cis-Retinsäure oder das Gemisch das 11,13-di-cis- und 13-cis-Isomer enthält, zu reinem 13-cis-Isomer isomerisiert. Die Isomerisierung wird in einem inerten Lösungsmittel durchgeführt, wobei als Katalysator eine Verbindung oder ein Komplex von Palladium oder Rhodium mit Ausnahme von Palladium- oder Rhodiumphthalocyanin oder einer einer Cyanidion enthaltenden Verbindung oder Komplex angewandt wird. Im allgemeinen wird die Reaktion bei Temperaturen von 10 bis 150 °C durchgeführt, wobei Temperaturen von 40 bis 65 °C, insbesondere 45 bis 55 °C besonders bevorzugt sind.

Erfindungsgemäss wurde gefunden, dass diese Katalysatoren die 11-cis-Doppelbindung selektiv in die entsprechende trans-Doppelbindung isomerisieren, ohne die 13-cis-Doppelbindung anzugreifen. Obschon einige Rhodium- oder Palladiumverbindungen oder Komplexe die 13-cis-Verbindung nicht in hohen Ausbeuten liefern, ist der Umstand, dass sie die 11-cis-Doppelbindung selektiv isomerisieren von grösster Bedeutung. In derartigen Fällen können die Ausbeuten dadurch verbessert werden, dass man die nicht umgewandelte 11-cis-Verbindung recyclisiert und erneut der Isomerisierung unterwirft. Wenn der Katalysator die 13-cis- zur 13-trans-Doppelbindung in irgendeinem Umfang isomerisieren würde, würde ein Ausbeuteverlust resultieren, weil die 13-trans-Doppelbindung nicht einfach isomerisiert werden kann. Da diese Katalysatoren in keiner Weise die 13-cis-Bindung angreifen, ist es möglich die Isomerisierung durchzuführen, ohne die 13-cis-Isomeren aus dem Reaktionsprodukt der Wittigreaktion zu isolieren. Auf diese Weise kann das Reaktionsprodukt der Isomerisierungsreaktion wiederholt der katalytischen Isomerisierung unterworfen werden, um die Ausbeute zu erhöhen, ohne dass das 13-cis-Isomer isoliert werden muss.

Jede der Verbindungen oder Komplexe von Palladium die in der US-A-4 051 174 beschrieben sind, können für die vorliegende Erfindung verwendet werden. Obschon ein homogenes katalytisches System bevorzugt ist, kann auch ein heterogenes katalytisches System verwendet werden. Im Falle der heterogenen katalytischen Isomerisierung kann der Katalysator in Abwesenheit eines Trägermaterials verwendet werden oder er kann auf einem Trägermaterial aufgebracht werden. Das Trägermaterial kann jedes übliche Trägermaterial sein, wie beispielsweise Kohle, Nickeloxid, Aluminiumoxid, Bariumsulfat, Calciumcarbonat und Molekularsiebe. Gewisse Polymere wie Nylon und Perlon können ebenfalls als Träger verwendet werden. Der Katalysator kann auf das Trägermaterial mit üblichen Verfahren aufgebracht werden.

Die Palladium- oder Rhodiumverbindungen oder Komplexe die als Katalysator in der vorliegenden Erfindung verwendet werden, sind vorzugsweise Salze oder Komplexe von Palladium oder Rhodium. Beispielsweise können die folgenden Palladiumsalze oder Komplexe verwendet werden: $PdCl_2$, $PdBr_2$, $PdF_2$, $PdI_2$, $K_2PdCl_4$, $PdOS_4$,
$K_2PdBr_4$, $(CH_3CN)_2PdCl_2$. $Pd(OAC)_2$,
(Benzonitril)$_2$PdCl$_2$, (Benzonitril)$_2$PdBr$_2$,
$(C_3H_5PdCl)_2$, (Cyclohexen-PdCl$_2$)$_2$,
(1,5-Cyclooctadien)PdCl$_2$,
(1,5-Cyclooctadien)PdBr$_2$,
(1,5-Cyclooctadien)PdI$_2$,
(Cyclooctatetraen)PdBr$_2$, (Arcylnitril)$_2$PdCl$_2$,
$Pd(NO_3)_4(NH_4)_2$, Pd(Pyridin)$_2$((NO$_2$)$_2$,
[N(CH$_2$)$_3$Benzyl]$_2$Pd(NO$_2$)$_4$, $Pd(NH_3)_2Cl_2$,
$Pd(NH_3)_2(NO_2)_2$, Pd(2,2-Bipyridyl)Cl$_2$,
$(NH_4)_2PdCl_4$, $(NH_3)_2PdCl_6$, $PdS_2$, $K_2PdCl_6$,
(Äthylendiamin)Pd(NO$_2$)$_3$,
(Amylamino)$_2$Pd(NO$_2$)$_2$, $(NH_3)_4Pd(NO_3)_2$,
Pd(Salicylaldoxim)$_2$, Bernsteinsäure-dinitril)PdCl$_2$, (Cyclooctatetraen)PdCl$_2$,
(Azobenzol)$_2$PdCl$_2$, (Bipyridyl)Pd(NO$_2$)$_2$,
K$_2$Pd(Malonat)$_2$, (Tricyclohexylphosphin)$_2$PdCl$_2$,
(Triphenylphosphin)$_2$PdCl$_2$, Tetrakis-(triäthylphosphit(Pd(O) und Tetrakis-(triphenylphosphin)Pd(O).

Die gleichen Salze oder Komplexe von Rhodium können ebenfalls verwendet werden.

Erfindungsgemäss wurde weiter gefunden, dass die katalytische Isomerisierung in hohen Ausbeuten eintritt ohne dass recyclisiert werden muss, wenn ein Katalysator verwendet wird, der durch Umsetzung eines Palladium(II)-salzes mit einem Tri(nieder-alkyl)- oder Tri(aryl)phosphin, vorzugsweise einem Tri(aryl)phosphin in Gegenwart eines Tri(nieder-alkyl)amins in einem inerten organischen Lösungsmittel, vorzugsweise Acetonitril erhalten wird. Die Anwendung eines Tri(nieder-alkyl)amins verbessert den Katalysator infolgedessen die bei der Isomerisierung erzielte Ausbeute.

Zur Erzielung bester Resultate ist die Verwendung von Palladium(II)nitrat als Palladiumsalz bevorzugt. Das bevorzugte Triarylphosphin ist Triphenylphosphin. Jedes übliche niedere Alkylamin kann zur Bildung des Komplexes verwendet werden, Triäthylamin ist bevorzugt.

Gemäss der bevorzugten Ausführungsform der vorliegenden Erfindung werden Palladium(II)nitrat und das Triarylphosphin in Acetonitril gelöst. Der Acetonitrillösung wird das Tri(nieder-alkyl)-amin zugesetzt, worauf der Katalysator als Niederschlag ausfällt. Gewünschtenfalls kann der Niederschlag abfiltriert werden und zur Isomerisierung des 11,13-di-cis-Isomeren entweder allein oder im Gemisch mit dem 13-cis-Isomeren verwendet werden. Andererseits kann die Lösung und der Niederschlag im Substrat zugesetzt werden. Zur Herstellung des bevorzugten Katalysators wird 1 Mol Palladium(II)nitrat mit mindestens 4 Mol Triarylphosphin umgesetzt. Man kann auch einen grösseren Überschuss von Triphenylphosphin verwenden. Da jedoch kein besonderer Vorteil in der Anwendung grosser Überschüsse von Triphenylphosphin besteht, werden Mengen von mehr als 10 Mol Triphenylphosphin pro Mol Palladiumsalz selten verwendet. Für die Umsetzung des Triphenylphosphins mit dem Palladium(II)nitrat kann jedes konventionelle Lösungsmittel für Triphenylphosphin und Palladium(II)nitrat angewandt werden. Die besten Resultate werden mit Acetonitril als Lösungsmittel erreicht. Bei der Verwendung eines Tri(nieder-alkyl)amins wie Triäthylamin zur Herstellung des Katalysators werden im allgemeinen mindestens 2 Mol Triäthylamin pro Mol Palladiumsalz oder Komplex angewandt. Gewünschtenfalls kann das Tri(nieder-alkyl)amin in grösseren Mengen wie 20 Mol pro Mol Palladiumsalz oder Komplex angewandt werden. Mit der Verwendung solcher grossen Mengen sind jedoch, wenn überhaupt, nur geringe Vorteile verbunden.

Bei der Durchführung der Isomerisierung ist der Katalysator in katalytischen Mengen anwesend. Im allgemeinen wird die Isomerisierung in einem inerten organischen Lösungsmittel durchgeführt. Jedes übliche inerte organische Lösungsmittel kann Verwendung finden. Bevorzugte Lösungsmittel sind Äther wie Tetrahydrofuran, Nitrile wie Acetonitril, und nieder-Alkylester von nieder-Alkancarbonsäuren, z. B. nieder-Alkancarbonsäure mit 2–4 C-Atomen wie Äthylacetat. Im allgemeinen ist es bevorzugt, dass der Katalysator in Mengen von etwa 0,0001 Mol bis etwa 0,01 Mol pro Mol 11,13-di-cis-Retinsäure anwesend ist. Der Katalysator kann im Überschuss angewendet werden in einer Menge von etwa 1 Mol pro Mol 11,13-di-cis-Retinsäure. Da jedoch keine Vorteile bei der Anwendung grosser Mengen Katalysator resultieren und diese Katalysatoren kostspielig sind, werden grosse Katalysatormengen selten verwendet. Im allgemeinen ist es bevorzugt, etwa 0,001 Mol bis etwa 0,01 Mol Katalysator pro Mol 11,13-di-cis-Retinsäure einzusetzen.

Nachdem die Isomerisierung durchgeführt ist, kann die 13-cis-Retinsäure aus dem Reaktionsgemisch in hohen Ausbeuten durch übliche Methoden wie Kristallisation gewonnen werden. Diese Kristallisation kann durch Zusatz von Wasser zum Reaktionsgemisch durchgeführt werden, wobei sich eine Suspension bildet, worauf man die Suspension auf eine Temperatur von 0 bis −5 °C kühlt.

Der hier verwendete Ausdruck «nieder Alkyl» bezieht sich auf geradkettige und verzweigte Alkylreste mit 1–7 Kohlenstoffatomen wie Methyl, Äthyl, Propyl und vorzugsweise Methyl. Nieder Alkoxy umfasst nieder Alkoxygruppen mit 1–7 C-Atomen wie Methoxy und Äthoxy. Nieder Alkancarbonsäuren haben 2–7 C-Atome wie Essigsäure, Propionsäure und Buttersäure. Halogen umfasst alle Halogene, nämlich Fluor, Chlor, Brom und Jod.

Der Ausdruck «Aryl» bezeichnet eine einkernige aromatische Kohlenwasserstoffgruppe wie Phenyl, die unsubstituiert oder in einer oder mehreren Stellungen durch nieder Alkylendioxy, Halogen, Nitro, nieder Alkyl oder nieder Alkoxy substituiert sein kann und mehrkernige Arylgruppen wie Naphthyl, Anthryl, Phenantrhyl und Azulyl, die durch eine oder mehrere der vorgenannten Gruppen substituiert sein können. Die bevorzugten Arylgruppen sind substituierte und unsubstituierte einkernige Arylgruppen, insbesondere Phenyl und Tolyl.

Die Erfindung wird durch die nachfolgenden Beispiele weiter erläutert.

Beispiel 1

Eine Lösung von 157,5 g [3-Methyl-5-(2,6,6-trimethyl-1-cyclohexen-1-yl)-2,4-pentadienyl]-triphenylphosphoniumchlorid (das Wittigsalz) und 57,0 g des Butenolids, 5-Hydroxy-4-methyl-2(5H)-furanon in 1000 ml Isopropanol wurde bei −30 °C unter Stickstoffatmosphäre gerührt. Die Lösung wurde mit 625 ml 2N wässrigem Kaliumhydroxyd in Isopropanol im Verlauf von 1 bis 1,5 Stunden bei einer Temperatur von −30 ±2 °C versetzt. Danach wurde das Reaktionsgemisch 10 Minuten gerührt und in 2500 ml Wasser gegossen. Die alkalische (pH 10) Lösung wurde mit zweimal 500 ml Hexan extrahiert. Die vereinigten Hexanextrakte wurden mit zweimal 100 ml Methanol-Wasser (7:3 Volumenteile) gewaschen und die Waschwässer wurden der ursprünglichen wässrigen Lösung zugesetzt. Die gesamte wässrige Lösung wurde durch Zusatz von 250 ml 4N wässriger Schwefelsäure angesäuert (pH 2) und mit 2000 ml und zweimal 1000 ml Äthylacetat-Hexan (2:8 Volumenteile) extrahiert. Die Äthylacetat-Hexan-Extrakte wurden einzeln nacheinander mit sechsmal 400 ml Methanol-Wasser (7:3 Volumenteile) gewaschen, wobei immer dieselbe Waschlösung verwendet wurde. Jede Methanol-Wasser-Waschlösung wurde nacheinander mit dreimal 1000 ml Äthylacetat-Hexan (2:8 Volumenteile) gewaschen, wobei immer die gleiche Waschlösung verwendet wurde. Zum Schluss erhielt man insgesamt sechs organische (Äthylacetat-Hexan)-Extrakte, und sechs wässrige (Methanol-Wasser)-Extrakte. Die Dünnschichtchromatographie zeigte, dass alles Triphenylphosphinoxyd in der wässrigen Phase war und dass die organischen Phasen 11,13-di-cis-Retinsäure und 16,7 Gew.-% 13-cis-Retinsäure enthielten. Die organischen Extrakte wurden vereinigt und mit siebenmal 500 ml Wasser gewaschen. Das Lösungsmittel wurde abgedampft und man erhielt 137,2 g (91,5%) eines Kristallgemischs, das 75,9 Gew.-% 11,13-di-cis-Retinsäure und 16,7 Gew.-% 13-cis-Retinsäure enthielt.

Beispiele 2–35

In den Beispielen 2–35, wurde das Wittigsalz und das Butenolid gemäss Beispiel 1 zu einem kristallinen Gemisch von 11,13-di-cis-Retinsäure und 13-cis-Retinsäure umgesetzt. Der durch die Anwendung verschiedener Temperaturen erzielte Effekt auf die Ausbeute ist in der nachstehenden Tabelle angegeben. Die Prozente sind auf Gewicht bezogen. In den Beispielen 2–23 und 26–35 war das Molverhältnis von Butenolid zu Wittigsalz wie in Beispiel 1. Wenn also die Menge Butenolid im Vergleich zu Beispiel 1 erhöht war, so war die Menge Wittigsalz im gleichen Masse erhöht, so dass dasselbe Molverhältnis wie in Beispiel 1 bestand. In Beispiel 24 wurde ein 10%iger molarer

Überschuss des Wittigsalzes im Vergleich zu Beispiel 1 angewandt. In Beispiel 25 wurde ein 20%iger molarer Überschuss des Wittigsalzes im Vergleich zu Beispiel 1 angewandt.

Tabelle 1

| Beispiel Nr. | Mole Butenolid | Temp. (°C) | % Ausbeute | Beispiel Nr. | Mole Butenolid | Temp. (°C) | % Ausbeute |
|---|---|---|---|---|---|---|---|
| 2 | 0.02 | 0 | 61.5 | 21 | 0.1 | –30 | 98.7 |
| 3 | 0.02 | 0 | 60.0 | 22 | 0.1 | –30 | 92.3 |
| 4 | 0.02 | –20 | 91.7 | 23 | 0.1 | –30 | 92.3 |
| 5 | 0.02 | 0 | 55.0 | 24 | 0.1 | –20 | 93.3 |
| 6 | 0.02 | –20 | 81.7 | 25 | 0.1 | 0 | 91.3 |
| 7 | 0.1 | –20 | 90.0 | 26 | 0.1 | –20 | 62.0 |
| 8 | 0.1 | –20 | 88.7 | 27 | 0.5 | –25 | 88.3 |
| 9 | 0.1 | –20 | 84.7 | 28 | 0.5 | –30 | 91.5 |
| 10 | 0.1 | –10 | 80.7 | 29 | 0.5 | –20 | 90.0 |
| 11 | 0.1 | –20 | 91.0 | 30 | 0.5 | –30 | 91.7 |
| 12 | 0.1 | –10 | 82.3 | 31 | 0.5 | –30 | 90.7 |
| 13 | 0.1 | –30 | 93.7 | 32 | 0.5 | –30 | 89.6 |
| 14 | 0.1 | –30 | 99.3 | 33 | 0.5 | –30 | 93.5 |
| 15 | 0.1 | –20 | 86.0 | 34 | 0.5 | –30 | 93.1 |
| 16 | 0.1 | –30 | 98.3 | 35 | 0.5 | –30 | 93.2 |
| 17 | 0.1 | –30 | 95.0 | | | | |
| 18 | 0.1 | –30 | 95.7 | | | | |
| 19 | 0.1 | –30 | 96.7 | | | | |
| 20 | 0.1 | –30 | 92.3 | | | | |

Beispiel 37

Eine Lösung von 137,2 g des in Beispiel 1 hergestellten Gemisches von Retinsäuren in 250 ml Tetrahydrofuran und 500 ml Acetonitril wurde unter Stickstoff und kräftigem Rühren auf 50 °C erwärmt. Ein Gemisch von 111 mg Palladium(II)nitrat, 509 mg Triphenylphosphin und 98 mg Triäthylamin in 25 ml Acetonitril wurde auf ein Mal zugegeben, worauf mit 25 ml Acetonitril nachgespült wurde. Das Gemisch wurde 1 Stunde auf 50 °C erwärmt. Unmittelbar nach Zusatz des Katalysators wurde die Reaktionslösung abgedunkelt. Innerhalb 1 Minute setzte die Kristallisation ein und innerhalb 2 Minuten resultierte eine dicke orange Suspension. Diese wurde unter Zusatz von 500 ml Wasser gekühlt, 2 Stunden bei 0 bis +5 °C gehalten und abfiltriert. Die Kristalle wurden mit viermal 100 ml kaltem Acetonitril-Wasser (25:75 Volumenteilen) gewaschen und bei Raumtemperatur unter vermindertem Druck getrocknet. Man erhielt 128,5 g 13-cis-Retinsäure (85,7 Gew.-% bezogen auf Botenolid).

Beispiele 38–43

In den Beispielen 38–43 wurde das kristalline Gemisch von Beispiel 1, das 75,9 Gew.-% 11,13-di-cis-Retinsäure und 16,7 Gew.-% 13-cis-Retinsäure enthielt, nach dem Verfahren von Beispiel 37 in 13-cis-Retinsäure umgewandelt, wobei andere Katalysatoren als Palladiumnitrat und Triphenylphosphin angewandt wurden. Dabei wurden die gleichen Bedingungen angewandt, mit der Ausnahme, dass alle Reaktionen 3 Stunden bei 50 °C durchgeführt wurden, mit 0,1 Mol-% Katalysator und 2 Äquivalenten Triäthylamin, bezogen auf den Katalysator. Die Isolierung der 13-cis-Retinsäure wurde durch Zusatz von überschüssigem Wasser (Lösungsmittelgehalt letztlich 62,5 Volumenprozent) und Filtration durchgeführt. Die Prozentangaben in Tabelle II beziehen sich auf das Gewicht der erhaltenen 13-cis-Retinsäure, bezogen auf das Gewicht des als Ausgangsmaterial eingesetzten kristallinen Gemisches.

Tabelle 2

| Beispiel Nr. | Katalysator | Gesamtausbeute | Analyse 13-cis | all-trans | 11,3-dicis |
|---|---|---|---|---|---|
| 38 | $(PhCN)_2PdCl_2$ | 97,0 | 90,3 | 4,5 | – |
| 39 | $(Ph_3P)_3RhCl$ | 97,3 | 58,1 | 1,8 | 35,1 |
| 40 | $(Ph_3P)_2PdCl_2$ | 98,0 | 20,6 | – | 74,5 |
| 41 | $(Ph_3P)RhH(CO)$ | 98,0 | 66,6 | 1,1 | 27,3 |
| 42 | $Pd(NO_3)_2 + Ph_3P$ | 97,3 | 91,0 | 0,6 | 4,1 |
| 43 | $(Ph_2P)_3RuCl_2$ | 93,3 | 15,4 | – | 78,9 |

Beispiel 44–53

Die Beispiele 44–53 wurden nach dem Verfahren von Beispiel 37 durchgeführt, wobei verschiedene Mengen Katalysator (Palladium(II)nitrat) zur Umwandlung des in Beispiel 1 erhaltenen kristallinen Gemischs von Retinsäuren in 13-cis-Retinsäure eingesetzt wurden.

Tabelle 3

| Beispiel Nr. | Mol Butenolid[a] | Katalysator Mol % | Reaktionsdauer Minuten | Ausbeute[b] (%) | Ausbeute[c] (%) | Reinheit |
|---|---|---|---|---|---|---|
| 44 | 0,1 | 0,047 | 30 | 78,1 | 70,3 | 97,2 |
| 45 | 0,1 | 0,074 | 30 | 83,6 | 78,3 | 94,0[d] |
| 46 | 0,1 | 0,043 | 30 | 91,4 | 85,3 | – |
| 47 | 0,1 | 0,070 | 150 | 78,5 | 78,0 | – |
| 48 | 0,1 | 0,046 | 180 | 84,4 | 83,0 | 97,0[e] |
| 49 | 0,1 | 0,10 | 30 | 86,8 | 81,0 | – |
| 50 | 0,5 | 0,10 | 60 | 93,1 | 82,3 | 92,7 |
| 51 | 0,5 | 0,10 | 60 | 93,7 | 85,7 | 100,5 |
| 52 | 0,5 | 0,10 | 60 | 94,2 | 84,8 | 99,4[f] |
| 53 | 0,5 | 0,10 | 60 | 94,5 | 86,7 | 99,8[g] |

[a] bei der Herstellung des Ausgangsmaterials gemäss Beispiel 1
[b] bezogen auf eingesetztes Isomerengemisch
[c] bezogen auf bei der Herstellung des Ausgangsmaterials eingesetztes Butenolid (nicht korrigiert)
[d] Produkt enthielt 1,4% 11,13-di-cis-Retinsäure
[e] Produkt enthielt 0,8% 11,13-di-cis-Retinsäure
[f] Produkt enthielt 0,7% all-trans- und 0,5% 11,13-di-cis-Retinsäure
[g] Produkt enthielt 0,7% 11,13-di-cis-Retinsäure

Beispiel 54

Alle Operationen wurden unter Stickstoff durchgeführt. In einem 190 l-Reaktor aus rostfreiem Stahl, der mit Trockeneis-Aceton gekühlt wurde und mit einer Turbinenvorrichtung versehen war, wurden 34,3 kg Isopropanol, 5,0 kg 5-Hydroxy-4-methyl-2(5H)-furanon und 23,0 kg [3-Methyl-5-(2,6,6-trimethyl-1-cyclohexen-1-yl)-2,4-pentadienyl]-triphenyl-phosphoniumchlorid eingefüllt. Das Gemisch wurde bis zur Lösung gerührt, auf −22 bis −25 °C gekühlt und bei dieser Temperatur gehalten. Danach wurden im Verlauf von 1,5 Stunden 49,9 kg einer 2,0N Kaliumhydroxyd-Isopropanol-Lösung (5–10 °C) bei −22 bis −25 °C zugesetzt. Danach wurde das Reaktionsgemisch eine weitere Stunde bei −22 bis −25 °C gerührt und zu 151,4 kg entionisiertem Wasser und 54,6 kg Hexan bei Raumtemperatur gegeben. Das Gemisch wurde 10 Minuten gerührt, absetzen gelassen und die untere Schicht (die das Material enthielt) abgetrennt. Die Hexanschicht wurde mit einem Gemisch von 4,8 kg Methanol und 2,6 kg entionisiertem Wasser extrahiert. Der Methanol-Wasser-Rückextrakt und die das Produkt enthaltende Phase wurde bei 0 bis +10 °C unter Stickstoff gehalten.

Das vorbeschriebene Verfahren wurde drei weitere Male durchgeführt, so dass in insgesamt vier Reaktionen 20,0 kg $C_5$-Butenolid eingesetzt wurden. Die vier Ansätze wurden vereinigt und auf ein pH von 4,0 bis 4,5 durch sorgfältigen Zusatz von 32,5–35,6 kg Gew.-%iger wässriger Phosphorsäure angesäuert. Das erhaltene Reaktionsgemisch wurde mit einem Gemisch von 115,8 kg Äthylacetat und 343,9 kg Hexan extrahiert (Extrakt Nr. 1).

Extrakt Nr. 1 wurde mit 94,6 kg entionisiertem Wasser und mit sechs Portionen eines Gemisches von 54,4 kg Methanol und 29,5 kg entionisiertem Wasser gewaschen. Jede der Waschflüssigkeiten wurde nacheinander mit fünf Portionen eines Gemisches von 44,2 kg Äthylacetat und 131,3 kg Hexan extrahiert. Die Äthylacetat-Hexan-Extrakte wurden vereinigt und bei einem Druck von 50–100 mmHg und einer Innentemperatur von 20–30 °C auf ein Endvolumen von etwa 570 l gebracht. Der Unterdruck wurde unter Stickstoff aufgehoben und 2,0 kg Kohle wurden zugesetzt. Der Ansatz wurde 30 Minuten bei 20–25 °C mit der Kohle gerührt und über einen Filter mit Diatomeenerde filtriert. Der Filterkuchen wurde mit 34,1 kg Äthylacetat gewaschen und das Waschwasser dem Ansatz zugesetzt. Der Ansatz wurde unter vermindertem Druck wie vorher auf ein Volumen von 95 l eingeengt. Danach wurde der Ansatz zu 34,1 kg Äthylacetat gegeben und erneut auf ein Volumen von 95 l gebracht. Die Temperatur des Ansatzes wurde auf 50 °C eingestellt, danach wurde eine Lösung von 2,65 kg Acetonitril, die 33,7 g Palladium(II)nitrat, 162,4 g Triphenylphosphin und 31,8 g Triäthylamin enthielten, gegeben. Die Lösung wurde dem Ansatz mit weiteren 1 l Äthylacetat zugefügt. Der Ansatz wurde dann bei 50 °C 1 Stunde gerührt, im Verlauf von 2 Stunden auf −10 bis −15 °C abgekühlt und über Nacht bei dieser Temperatur gehalten. Die Kristalle von roher 13-cis-Retinsäure wurden abfiltriert und mit dreimal 10,2 kg Äthylacetat bei −10 bis −15 °C gewaschen. Die Kristalle wurden soweit wie möglich in der Zentrifuge trocken geschleudert. 356,4 kg Äthylacetat und 39,7 kg Äthylacetatfeuchte 13-cis-Retinsäure und 2,0 kg Kohle wurden zum Rückfluss (75–80 °C) erhitzt. Das Gemisch wurde 30 Minuten am Rückfluss erhitzt. Die Lösung wurde durch ein Filter mit Diatomeenerde unter Druck filtriert. Das Filter wurde mit 68,1 kg Acetylacetat gewaschen und das Waschwasser dem Ansatz zugesetzt. Der Ansatz wurde unter Stickstoff auf ein Volumen von 11,5 l bei einer Innentemperatur von 75–80 °C eingeengt. Danach wurde auf − 10 bis − 15 °C gekühlt und bei dieser Temperatur über Nacht stehen gelassen. Die erhaltenen Kristalle der reinen 13-cis-Retinsäure wurden abfiltriert und mit drei Portionen 10,2 kg Äthylacetat bei − 10 bis − 15 °C gewaschen. Die Kristalle wurden 24 Stunden bei einem Druck von 50–100 mmHg bei 35 °C unter Stickstoff getrocknet.

Beispiel 55

Eine Lösung von 262,5 g [3-Methyl-5-(2,6,6-trimethyl-2-cyclohexen-1-yl)-2,4-pentadienyl]-triphenylphosphoniumchlorid, 57,0 g $C_5$-Butenolid (0,500 Mol) und 1000 ml Isopropanol wurde unter Stickstoff auf − 25 °C gekühlt und im Verlauf von 1 Stunde bei einer Temperatur von − 20 bis − 25 °C mit 625 ml 2,0N Kaliumhydroxyd-Isopropanol (1,25 Mol) versetzt. Danach wurde das Gemisch eine weitere Stunde bei − 25 °C gerührt und dann in 2500 ml entionisiertes Wasser gegossen. Das erhaltene Gemisch wurde mit zweimal 500 ml Hexan extrahiert, um unpolare Verunreinigungen zu entfernen. Die vereinigten Hexanschichten wurden mit zweimal 100 ml Methanol-Wasser (70–30 Volumenprozent) gewaschen. Die vereinigten wässrigen Phasen wurden durch Zusatz von 55 ml 85%iger Phosphorsäure auf pH 4 gebracht und die erhaltene Suspension mit 2000 ml Äthylacetat-Hexan (20–80 Volumenprozent) extrahiert. Dieser Extrakt wurde nacheinander mit sechsmal 400 ml Methanol-Wasser (70–30 Volumenprozent) gewaschen. Jede Waschflüssigkeit wurde getrennt aufbewahrt und nacheinander mit fünfmal 1000 ml Äthylacetat-Hexan (20–80 Volumenprozent) rückextrahiert. Danach wurden die sechs Äthylacetat-Hexan-Extrakte kombiniert, mit zweimal 500 ml entionisiertem Wasser gewaschen und unter vermindertem Druck zu 130,4 g (86,9% Ausbeute) eines kristallinen Gemisches konzentriert, das etwa 70–85 Gew.-% 11,13-di-cis-Retinsäure und etwa 15–30 Gew.-% 13-cis-Retinsäure enthielt.

Beispiel 56

130,4 g des in Beispiel 55 hergestellten kristallinen Gemischs und 200 ml Äthylenacetat wurden bei 50 °C unter Stickstoff gerührt und ein Gemisch von 100 mg Palladium(II)nitrat, 482 mg Triphenylphosphin, 94,4 mg Triäthylamin und 25 ml Acetonitril wurde zugesetzt. Das Reaktionsgemisch wurde sogleich abgedunkelt und kristallisierte innerhalb 1 Minute zu einer dicken orangen Suspension. Dieses Gemisch wurde 1 Stunde bei 50 °C gerührt, 2 Stunden auf − 10 bis − 15 °C gekühlt, abfiltriert, der Niederschlag mit dreimal 50 ml kaltem Äthylacetat gewaschen und unter vermindertem Druck getrocknet. Man erhielt 117,7 g reine 13-cis-Retinsäure (90,3% Ausbeute des in Beispiel 55 hergestellten kristallinen Gemisches, 78,4% Gesamtausbeute bezogen auf $C_5$-Butenolid).

Beispiel 57

121,2 g des in Beispiel 55 erhaltenen kristallinen Gemisches von 13-cis-Retinsäure und 11,13-di-cis-Retinsäure wurden in 500 ml Diäthyläther gelöst. Aus dieser Lösung wurden 250 ml Äther abdestilliert, die durch ein gleiches Volumen Hexan ersetzt wurden. Weitere 250 ml Hexan wurden zugesetzt und die erhaltene Lösung wurde auf Raumtemperatur abkühlen gelassen und etwa 4 Stunden ruhig stehen gelassen. Die Kristalle wurden abfiltriert, mit dreimal 100 ml kaltem Hexan gewaschen und bei Raumtemperatur unter vermindertem Druck getrocknet. Drei solcher Ansätze wurden vereinigt und das obige Umkristallisationsverfahren wurde zweimal wiederholt. Aus drei Umkristallisationen aus Äther-Hexan wurde 74,6 kg kristalline 11,13-di-cis-Retinsäure erhalten. Diese wurde unter Erwärmen in 250 ml Äthylacetat gelöst und die warme Lösung (55–60 °C) wurde durch Diatomeenerde filtriert, um Fremdstoffe zu entfernen. Der Filterkuchen wurde mit zweimal 100 ml heissem (75–80 °C) Äthylacetat gewaschen. Die vereinigten Filtrate und Waschflüssigkeiten wurden auf ein Volumen von 200 ml gebracht und wie oben beschrieben abkühlen gelassen. Die gebildeten Kristalle wurden filtriert, mit zweimal 100 ml kaltem Äthylacetat gewaschen und unter vermindertem Druck getrocknet. Man erhiel 39,3 g reine 11,13-di-cis-Retinsäure vom Schmelzpunkt 124,5–126 °C.

Beispiel 58

Eine Lösung von 8,0 g 11,13-di-cis-Retinsäure und 25 ml Äthylacetat wurde unter Stickstoff mit einem Gemisch von 10,0 mg Palladium(II)nitrat, 60 mg Triphenylphosphin, 9,4 mg Triäthylamin und 2,5 ml Acetonitril versetzt. Kristallisation trat innerhalb 3–5 Minuten ein. Das Gemisch wurde bei 50 °C 1 Stunde gerührt, auf − 10 °C gekühlt und filtriert. Die Kristalle wurden mit dreimal 20 ml kaltem (− 20 °C) Äthylacetat gewaschen und unter vermindertem Druck getrocknet. Man erhielt 7,28 g (91,5%) 13-cis-Retinsäure.

**Patentansprüche**

1. Verfahren zur Herstellung von 13-cis-Retinsäure, dadurch gekennzeichnet, dass man

a) 5-Hydroxy-4-methyl-2(5H)-furanon mit einem Salz der Formel

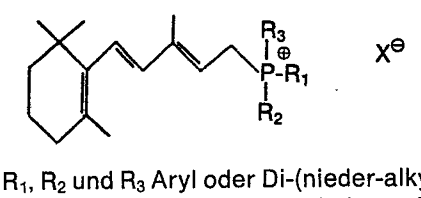

I

worin $R_1$, $R_2$ und $R_3$ Aryl oder Di-(nieder-alkyl)amino und X Halogen sind, in einem niederen Alkanol in Gegenwart eines Alkalimetallhydroxids bei einer Temperatur von − 10 bis − 50 °C umsetzt, und

b) das in Stufe a) erhaltene Reaktionsprodukt in einem organischen Lösungsmittel mit einer Verbindung oder einem Komplex von Rhodium oder Palladium, ausgenommen Palladium- oder Rhodiumphthalocyanin oder einer ein Cyanidion enthaltenden Palladium- oder Rhodiumverbindung behandelt.

2. Verfahren nach Anspruch 1, dadurch gekennzeichnet, dass die Reaktion bei −20 bis −50 °C durchgeführt wird.

3. Verfahren nach Anspruch 1, dadurch gekennzeichnet, dass die Reaktion bei −30 bis −45 °C ausgeführt wird.

4. Verfahren nach Anspruch 1, dadurch gekennzeichnet, dass der Katalysator durch Umsetzung von Palladium(II)nitrat mit einem Triaryl- oder Trialkylphosphin in Gegenwart eines niederen Alkylamins in einem Lösungsmittel hergestellt wird.

5. Verfahren nach Anspruch 4, dadurch gekennzeichnet, dass das Phosphin Triphenylphosphin und das Amin Diäthylamin ist.

6. Verfahren nach Anspruch 5, dadurch gekennzeichnet, dass das Lösungsmittel Acetonitril ist.

7. Verfahren zur Herstellung von 13-cis-Retinsäure, dadurch gekennzeichnet, dass man 11,13-di-cis-Retinsäure in einem inerten organischen Lösungsmittel mit einem Katalysator behandelt, wobei der Katalysator eine Verbindung oder einen Komplex von Rhodium oder Palladium ausgenommen Palladium- oder Rhodiumphthalocyanin oder eine ein Cyanidion enthaltende Palladium- oder Rhodiumverbindung ist.

8. Verfahren zur Herstellung eines Gemisches, das 13-cis-Retinsäure und 11,13-di-cis-Retinsäure enthält, dadurch gekennzeichnet, dass man 5-Hydroxy-4-methyl-2(5H)-furanon mit einem Salz der Formel

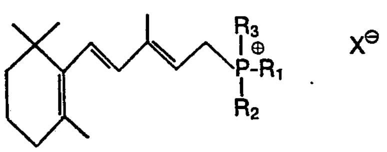

I

worin $R_1$, $R_2$ und $R_3$ Aryl oder Di-(nieder-alkyl)amino und X Halogen sind, in einem niederen Alkanol in Gegenwart eines Alkalimetallhydroxids bei einer Temperatur von −10 bis −50 °C umsetzt.

9. Verfahren nach Anspruch 8, dadurch gekennzeichnet, dass die Reaktion in Isopropanol in Gegenwart eines Alkalimetallhydroxids durchgeführt wird.

10. Verfahren zur Herstellung eines Palladiumkatalysators, dadurch gekennzeichnet, dass man 1 Mol eines Palladium(II)salzes ausgenommen Palladium(II)cyanid oder Palladium(II)phthalocyanin mit mindestens 4 Molen eines Triarylphosphins in einem organischen Lösungsmittel umsetzt und danach ein Tri-(nieder-alkyl)amin in einer Menge von mindestens 2 Molen Amin pro Mol Palladium(II)salz zusetzt.

11. Verfahren nach Anspruch 10, dadurch gekennzeichnet, dass das Triarylphosphin in einer Menge von 4–10 Mol pro Mol Palladium(II)salz und das Tri-(nieder-alkyl)amin in einer Menge von 2–20 Molen pro Mol Palladium(II)salz anwesend ist.

12. Verfahren nach Anspruch 10, dadurch gekennzeichnet, dass das Palladium(II)salz Palladium(II)nitrat und das Phosphin Triphenylphosphin ist.

13. Verfahren nach Anspruch 12, dadurch gekennzeichnet, dass das Lösungsmittel Acetonitril ist.

14. Verfahren nach Anspruch 13, dadurch gekennzeichnet, dass das Amin Triäthylamin ist.

**Claims**

1. A process for the manufacture of 13-cis-retinoic acid, characterized by

a) reacting 5-hydroxy-4-methyl-2(5H)-furanone with a salt of the formula

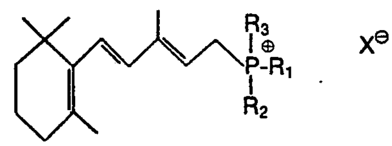

I

wherein $R_1$, $R_2$ and $R_3$ are aryl or di-(lower-alkyl)-amino and X is halogen, in a lower alkanol in the presence of an alkali metal hydroxide at a temperature of −10 to −50 °C, and

b) treating the reaction product obtained in step a) in an organic solvent with a compound or a complex of rhodium or palladium other than palladium or rhodium phthalocyanine or a palladium or rhodium compound containing a cyanide ion.

2. A process according to claim 1, characterized in that the reaction is carried out at −20 to −50 °C.

3. A process according to claim 1, characterized in that the reaction is carried out at −30 to −45 °C.

4. A process according to claim 1, characterized in that the catalyst is prepared by reacting palladium(II)nitrate with a triarylphosphine or trialkylphosphine in the presence of a lower alkylamine in a solvent.

5. A process according to claim 4, characterized in that the phosphine is triphenylphosphine and the amine is diethylamine.

6. A process according to claim 5, characterized in that the solvent is acetonitrile.

7. A process for the manufacture of 13-cis-retinoic acid, characterized by treating 11,13-di-cis-retinoic acid in an inert organic solvent with a catalyst, the catalyst being a compound or a complex of rhodium or palladium other than palladium or rhodium phthalocyanine or a palladium or rhodium compound containing a cyanide ion.

8. A process fo the manufacture of a mixture which contains 13-cis-retinoic acid and 11,13-di-cis-retinoic acid, characterized by reacting 5-hydroxy-4-methyl-2(5H)-furanone with a salt of the formula

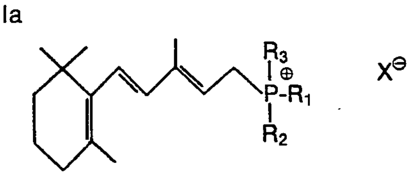

I

wherein $R_1$, $R_2$ and $R_3$ are aryl or di-(lower-alkyl)-amino and X is halogen, in a lower alkanol in the presence of an alkali metal hydroxide at a temperature of −10 to −50 °C.

9. A process according to claim 8, characterized in that the reaction is carried out in isopropanol in the presence of an alkali metal hydroxide.

10. A process for the production of a palladium catalyst, characterized by reacting 1 mol of a palladium(II) salt other than palladium(II) cyanide or palladium(II) phthalocyanine with at least 4 mols of a triarylphosphine in an organic solvent and thereafter adding a tri-(lower-alkyl)amine in an amount of at least 2 mols of amine per mol of palladium(II) salt.

11. A process according to claim 10, characterized in that the triarylphosphine is present an amount of 4–10 mol per mol of palladium(II) salt and the tri-(lower-alkyl)amine is present in an amount of 2–20 mols per mol of palladium(II) salt.

12. A process according to claim 10, characterized in that the palladium(II) salt is palladium(II) nitrate and the phosphine is triphenylphosphine.

13. A process according to claim 12, characterized in that the solvent is acetonitrile.

14. A process according to claim 13, characterized in that the amine is triethylamine.

**Revendications**

1. Procédé de préparation d'acide 13-cis-rétinoïque, caractérisé en ce que

a) On fait réagir de la 5-hydroxy-4-méthyl-2(5H)-furanone avec un sel de formule

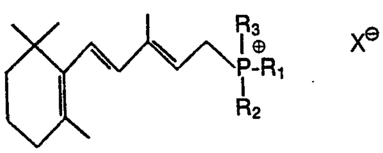

I

où $R_1$, $R_2$ et $R_3$ représentent un aryle ou un di-(alcoyle inférieur)amino et X un halogène, dans un alcanol inférieur en présence d'un hydroxyde de métal alcalin à une température allant de −10 à −50 °C, et

b) on traite le produit réactionnel obtenu dans l'étape a) dans un solvant organique avec un composé ou un complexe de rhodium ou de palladium, à l'exception de la palladium- ou rhodium-phthalocyanine, ou un composé de palladium ou de rhodium contenant une cyanidione.

2. Procédé selon la revendication 1, caractérisé en ce que la réaction est conduite entre −20 et −50 °C.

3. Procédé selon la revendication 1, caractérisé en ce que la réaction est conduite entre −30 et −45 °C.

4. Procédé selon la revendication 1, caractérisé en ce que le catalyseur est préparé par réaction de nitrate de palladium (II) avec une triaryl- ou trialcoylphosphine en présence d'une alcoylamine inférieure dans un solvant.

5. Procédé selon la revendication 4, caractérisé en ce que la phosphine est la triphénylphosphine et l'amine la diéthylamine.

6. Procédé selon la revendication 5, caractérisé en ce que le solvant est l'acétonitrile.

7. Procédé de préparation d'acide 13-cis-rétinoïque, caractérisé en ce qu'on traite l'acide 11,13-di-cis-rétinoïque dans un solvant organique inerte avec un catalyseur, où le catalyseur est un composé ou un complexe de rhodium ou de palladium à l'exception de la palladium- ou rhodium-phthalocyanine, ou un composé de palladium ou de rhodium contenant une cyanidione.

8. Procédé de préparation d'un mélange qui contient l'acide 13-cis-rétinoïque et l'acide 11,13-di-cis-rétinoïque, caractérisé en ce qu'on fait réagir la 5-hydroxy-4-méthyl-2(5H)-furanone avec un sel de formule

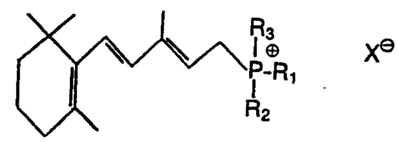

I

où $R_1$, $R_2$ et $R_3$ représentent un aryle ou une di-(alcoyle inférieure)amino et X un halogène, dans un alcanol inférieur en présence d'un hydroxyde de métal alcalin à une température allant de −10 à −50 °C.

9. Procédé selon la revendication 8, caractérisé en ce que la réaction est conduite dans l'isopropanol en présence d'un hydroxyde de métal alcalin.

10. Procédé de préparation d'un catalyseur à base de palladium, caractérisé en ce qu'on fait réagir une mole d'un sel de palladium (II) à l'exception du cyanure de palladium (II) ou de l phthalocyanine de palladium (II) avec au moins 4 moles d'une triarylphosphine dans un solvant organique puis en ce qu'on ajoute une tri-(alcoyle inférieure)amine en une quantité d'au moins 2 moles d'amine par mole de sel de palladium (II).

11. Procédé selon la revendication 10, caractérisé en ce que la triarylphosphine est présente en une quantité de 4–10 moles par mole de sel de palladium(II) et la tri-(alcoyle inférieure)amine en une quantité de 2–20 moles par mole de sel de palladium(II).

12. Procédé selon la revendication 10, caractérisé en ce que le sel de palladium(II) est le nitrate de palladium(II) et en ce que la phosphine est la triphénylphosphine.

13. Procédé selon la revendication 12, caractérisé en ce que le solvant est l'acétonitrile.

14. Procédé selon la revendication 13, caractérisé en ce que l'amine est la triéthylamine.